# EUROPEAN PATENT APPLICATION

(11) **EP 2 617 721 A2**
(43) Date of publication of application: **24.07.2013**
(21) Application number: 11820192.0
(22) Date of filing: 25.08.2011
(51) Int. Cl.: C07D 471/04, C07D 401/06, A61K 31/437, A61P 17/00

(54) **NOVEL HETEROCYCLIC COMPOUND, AND COMPOSITION FOR TREATING INFLAMMATORY DISEASES USING SAME**

(30) Priority: 25.08.2010 KR 20100082258
(71) Applicant: Neopharm Co., Ltd., Daejeon 305-510 (KR)
(72) Inventor: GWAK, Hyung Sub, Daejeon 305-510 (KR); KIM, Yong Zu, Daejeon 305-811 (KR); KIM, Uk Il, Daejeon 305-811 (KR); PARK, Byeong Deog, Daejeon 305-510 (KR); PARK, Tae Kyo, Daejeon 305-811 (KR); WOO, Sung Ho, Daejeon 305-811 (KR); YUN, Joung Yul, Daejeon 305-811 (KR); LEE, Dae Yon, Daejeon 305-811 (KR); LEE, Hyang Sook, Daejeon 305-811 (KR); JEON, Jeong Eun, Daejeon 305-510 (KR); JEONG, Se Kyoo, Daejeon 305-510 (KR); CHOI, Hyung Mook, Daejeon 305-510 (KR)
(74) Representative: Stolmár & Partner
(86) International application number: PCT/KR2011/006278
(87) International publication number: WO 2012/026765

(57) **Abstract**

The heterocyclic compound according to the present invention may treat and prevent inflammatory diseases and heal skin wounds, and particularly, have effects of recovering damaged skin barrier, reducing inflammation and pruritus in inflammatory skin diseases. In addition, the composition containing the compound according to the present invention as an active ingredient may be used to alleviate various inflammatory diseases and diseases in which PAR-2 is excessively expressed, and particularly, the composition may inhibit activity of PAR-2 in skin, such that the composition may be used as a composition having anti-inflammatory function in inflammatory skin diseases including atopic dermatitis, or the like.

## Description

### [Technical Field]

The present invention relates to a protease activated receptor-2 (PAR-2) inhibitor and a composition for treating and preventing an inflammatory disease, as compounds containing imidazopyridine derivatives, which are novel heterocyclic compounds, and salts thereof.

### [Background Art]

Protease activated receptor-2 (PAR-2), which is a receptor belonging to a G-protein-coupled receptor (GPCR), is a receptor found in association with a thrombin receptor (PAR-1) discovered in 1991. As a result of research into PAR-2 activation mechanism, it is known that the PAR-2 has a specific activation mechanism in that PAR-2 is activated as a peptide sequence (SLIGRL in human) exhibited in a receptor terminal is bonded to a specific site of the PAR-2 while protease such as trypsin or mast cell-derived tryptase decomposes a specific site of peptide sequences present in a terminal of an extracellular domain of the PAR-2 (Exp. Rev. Mol. Med., 4(16), 1-17, 2002).

Recently, it is reported that the PAR-2 plays important roles in inflammatory response of skin, a skin barrier function, generation of pruritus, or the like, relationship between the role of the PAR-2 and atopic dermatitis having the above-mentioned symptoms as main symptoms has been strongly suggested. It was known that the PAR-2 is expressed in various cells of human, and particularly, it was reported that the PAR-2 induces an inflammatory response and an activation reaction of nerve cells, or the like. In addition, it was reported that since the PAR-2 stimulates movement of melanosome while being involved in a signal transmission mechanism between kerationocyte and melanocyte in the skin, the PAR-2 is closely associated with skin pigmentation (Drug Dev. Res., 59, 408-416, 2003).

In addition, with regard to the skin barrier function, it was reported that activation of the PAR-2 is closely associated with the skin barrier function. That is, it was reported that when the skin barrier function is damaged, an activity of the protease in a stratum corneum is rapidly increased and thus the PAR-2 is activated (J. Invest. Dermatol., 126, 2074-2076, 2006), and it was confirmed that a material derived from dust mite or a cockroach, which is a kind of allergens inducing and exacerbating atopic dermatitis, also may activate the PAR-2. According to a prior study of the present inventors, in the case in which an allergen was applied on skin barrier-damaged skin of which a skin barrier was damaged, the recovery of the skin barrier function was hindered due to activation of the PAR-2 as described above, and in the case in which the allergen and a PAR-2 inhibitor were simultaneously applied, a barrier recovery mechanism was normalized (J. Invest. Dermatol., 128, 1930-1939, 2008). Further, it was reported that when the PAR-2 inhibitor is applied on the skin barrier-damaged skin, the recovery of the skin barrier is stimulated. It is expected that since damage of the skin barrier in the skin of the atopic dermatitis patient is the most general symptom, the recovery effect of the PAR-2 inhibitor from the skin barrier damage will assist in treating symptoms of atopic dermatitis.

It was reported that when the PAR-2 is activated in the skin, pruritus is generated, and a behavior of scratching the skin is induced due to this pruritus. According to an existing study results on an anti-pruritus effect of the PAR-2 inhibitor (JP 2004-170323), it was reported that in the case in which the PAR-2 inhibitor is applied to the skin, scratching is significantly decreased. As a mechanism of pruritus, it is known that nerve cells are activated by activation of the PAR-2 present in nerve fibers in the skin, and then a pruritus signal is transmitted to a brain. A function of PAR-2 associated with pruritus was confirmed through the fact that in the case of applying a PAR-2 activating material to the skin of animal models, the number of scratching action was rapidly increased, and in the case of inhibiting the activity of PAR-2, this phenomenon was suppressed (J. Neurosci., 23, 6176-6180, 2003).

As a material having a selective PAR-2 inhibitory effect, it is known that currently, two kinds are developed all over the word. Among them, a material of which the effect is verified through academic journals is an ENMD-1,068 produced by ENtreMed Corp. which is a bio-venture company in the US. Further, a PAR-2 inhibitor produced by Sumitomo Pharma Co. is disclosed in Japanese Patent Laid- open Publication No. 2004-170323.

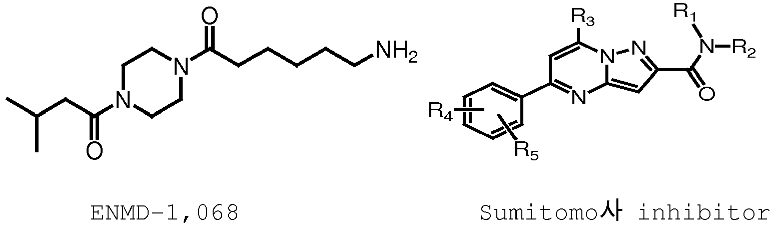

Exacerbation of inflammation by the PAR-2 and an anti-inflammatory effect of a material having a PAR-2 inhibitory effect was confirmed through the fact that when trypsin, which is a protease activating PAR-2, or activating peptide (AP), which is an activating material of PAR-2, is applied to the skin, the inflammatory response is increased, and when the protease inhibitor is simultaneously applied, this inflammatory response is decreased (FASEB. J., 17, 1871-1885, 2003).

The present inventors synthesized various materials and confirmed the activity thereof over a long time in order to synthesize a material having PAR-2 inhibitory activity. As a result, the present inventors developed a novel compound having excellent PAR-2 inhibitory activity.

### [Disclosure]

### [Technical Problem]

An object of the present invention is to provide imidazopyridine compounds represented by the following Chemical Formula 1, as a novel heterocyclic compound.

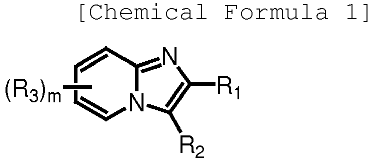

Another object of the present invention is to provide a pharmaceutical composition containing compounds of Chemical Formula 1, which are the heterocyclic compounds according to the present invention, and pharmaceutically acceptable salts thereof, for treating or preventing an inflammatory disease, and provide a protease activated receptor-2 (PAR-2) inhibitor composition containing the compounds of Chemical Formula 1 or pharmaceutically acceptable salts thereof.

### [Technical Solution]

In one general aspect, according to the present invention, there is provided imidazopyridine compounds represented by the following Chemical Formula 1, which are novel heterocyclic compounds.

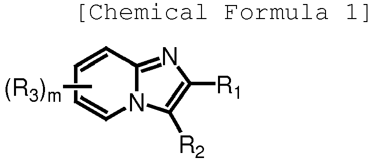

[In Chemical Formula 1,
R₁ is selected from hydrogen, (C1-C7)alkyl, (C3-C6)cycloalkyl, (C6-12)aryl, (C6-C12)ar(C1-C7)alkyl, and 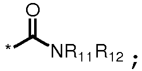 R₂ and R₃ each are independently selected from hydrogen, halogen, (C1-C7)alkyl, (C1-C7)alkoxy, (C1-C7)alkoxycarbonyl, (C3-C6)cycloalkyl, (C6-C10)aryl, (C6-C10)ar(C1-C7)alkyl, (C1-C7) alkylamido(C1-C7)alkyl, -NR₁₁R₁₂, 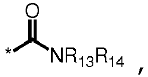 and 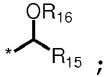 R₁₁, R₁₂, R₁₃, R₁₄, and R₁₅ each are independently selected from hydrogen, (C1-C7)alkyl, (C3-C6)cycloalkyl, (C6-C10)aryl, heterocycloalkyl(C6-C12)aryl, and heteroaryl; R₁₁ and R₁₂, and R₁₃ and R₁₄ are linked to each other via alkylene, oxyalkylene, or aminoalkylene to form 5- or 6-membered heterocyclic ring; R₁₆ is selected from hydrogen, (C1-C7)alkyl, (C3-C6)cycloalkyl, or (C6-C10)aryl;
m is an integer of 1 to 4; and
(C6-C12)aryl of R₂ and R₃ and heterocyclic ring formed from ; R₁₁ and R₁₂, and R₁₃ and R₁₄ may be further substituted with halogen, hydroxyl, (C1-C7)alkyl, (C3-C6)cycloalkyl, 5- or 6-membered heterocycloalkyl, 5- or 6-membered heteroaryl, 5- or 6-membered heterocycloalkylcarbonyl, (C6-C10)aryl (C6-C10)arylcarbonyl, (C1-C6)alkoxy, (C1-C7)alkoxycarbonyl, (C1-C7)alkylcarbonyl, (C3-C6)cyclo(C1-C7)alkylcarbonyl, (C6-C10)ar(C1-C7)alkylcarbonyl, (C3-C6)heteroarylcarbonyl, 5- or 6-membered cycloalkylsulfonyl, (C1-C7)alkylsulfonyl, amino(C1-C7)alkylcarbonyl, (C1-C7)alkyloxycarbonyl, (C6-C10)ar(C1-C7)alkyloxycarbonyl, amino, (C1-C7)alkylamino, (C1-C7)alkylamido, (C1-C7)alkylcarbamoyl, (C1-C7)alkylsulfonamido, or 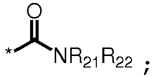 and R₂₁ and R₂₂ each may be independently (C1-C7)alkyl, (C3-C6)cycloalkyl, or (C6-C12)ary, or R₂₁ and R₂₂ may be linked to each other via alkylene to form 5- or 6-membered heterocyclic ring.]

In the present invention, the terms "alkyl", "alkoxy" and other substituents including an "alkyl" part may include both of the straight chain type and the branched chain type. In addition, (C1-C7) alkyl includes methyl, ethyl, n-propyl, i-propyl, n-butyl, i-butyl, t-butyl, n-pentyl, i-pentyl, n-hexyl, and n-heptyl; (C1-C6)alkoxy includes methoxy, ethoxy, n-propoxy, i-propoxy, n-butoxy, i-butoxy, t-butoxy, n-penthoxy i-penthoxy, and n-hexyloxy; (C3-C6)cycloalkyl includes cyclopropyl, cyclobutyl, cyclopentyl, and cyclohexyl; and (C6-C12)aryl includes phenyl, naphthyl, biphenyl, and anthryl. 5- or 6-membered heterocyclic ring may include both of the aliphatic heterocyclic ring and heteroaryl. Particularly, heterocycloalkyl, that is, aliphatic heterocyclic ring may include morpholinyl, thiomorpholinyl, pyrrolidinyl, piperidinyl, piperazinyl, pyrrolidonyl, piperidonyl, oxazolidinonyl, thiazolidinoyl; heteroaryl includes monocyclic heteroaryl such as furyl, thiophenyl, pyrrolyl, pyranyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl, or the like, and polycyclic heteroaryl such as benzofuranyl, benzothiophenyl, isobenzofuranyl, benzimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinolizinyl, quinoxalinyl, carbazolyl, phenanthridinyl, benzodioxolyl, or the like.

The compound of Chemical Formula 1, which is the heterocyclic compound according to the present invention, may include imidazopyridine compounds represented by the following Chemical Formula 2. 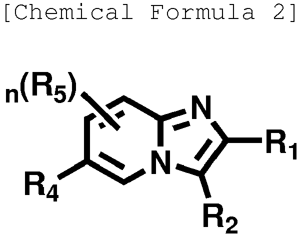 R₁ is selected from hydrogen, (C1-C7)alkyl, (C3-C6)cycloalkyl, (C6-12)aryl, and 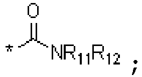 R₂ is independently selected from hydrogen, halogen, amino, (C1-C7)alkyl, (C6-10)aryl, -NR₁₁R₁₂, 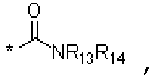 and 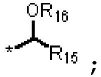 R₄ may be selected from hydrogen, halogen, (C1-C7)alkyl, (C1-C7)alkoxy, (C1-C7)alkoxycarbonyl, (C6-C10)aryl, (C1-C7)alkylamido(C1-C7)alkyl, -NR₁₁R₁₂, 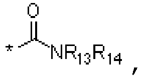 and 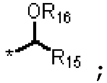 R₁₁, R₁₂, R₁₃, R₁₄, and R₁₅ each are independently selected from hydrogen, (C1-C7)alkyl, (C3-C6)cycloalkyl, (C6-C10)aryl, heterocycloalkyl(C6-C12)aryl, and heteroaryl; R₁₁ and R₁₂, and R₁₃ and R₁₄ may be bonded to alkylene, oxyalkylene, or aminoalkylene to form a 5- or 6-membered heterocyclic ring; R₁₆ may be selected from hydrogen, (C1-C7)alkyl, (C3-C6)cycloalkyl, or (C6-C10)aryl;
(C6-C12)aryl of R₂ and R₄ and heterocyclic ring formed from R₁₁ and R₁₂, and R₁₃ and R₁₄ may be further substituted with halogen, hydroxyl, (C1-C7)alkyl, (C3-C6)cycloalkyl, 5- or 6-membered heterocycloalkyl, 5- or 6-membered heteroaryl, 5- or 6-membered heterocycloalkylcarbonyl, (C6-C10)aryl (C6-C10)arylcarbonyl, (C1-C6)alkoxy, (C1-C7)alkoxycarbonyl, (C1-C7)alkylcarbonyl, (C3-C6)cyclo(C1-C7)alkylcarbonyl, (C6-C10)ar(C1-C7)alkylcarbonyl, (C3-C6)heteroarylcarbonyl, 5- or 6-membered cycloalkylsulfonyl, (C1-C7)alkylsulfonyl, amino(C1-C7)alkylcarbonyl, (C1-C7)alkyloxycarbonyl, (C6-C10)ar(C1-C7)alkyloxycarbonyl, amino, (C1-C7)alkylamino, (C1-C7)alkylamido, (C1-C7)alkylcarbamoyl, (C1-C7)alkylsulfonamido, or 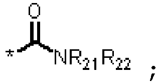 R₂₁ and R₂₂ each may be independently (C1-C7)alkyl, (C3-C6)cycloalkyl, or (C6-C12)ary, or R₂₁ and R₂₂ may be linked to each other via alkylene to form 5- or 6-membered heterocyclic ring;
R₅ may be independently selected from halogen and (C1-C7)alkyl; and
n is an integer of 1 to 3.]

In addition, the compound of Chemical Formula 1, which is the heterocyclic compound according to the present invention, may include imidazopyridine compounds represented by the following Chemical Formula 3.

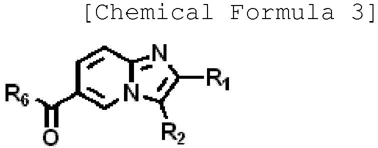

[In Chemical Formula 3,
R₁ is selected from hydrogen, (C1-C7)alkyl, (C3-C6)cycloalkyl, (C6-12)aryl, and 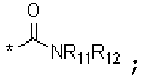 R₂ is independently selected from hydrogen, halogen, amino, (C1-C7)alkyl, (C6-10)aryl, -NR₁₁R₁₂, 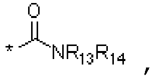 and 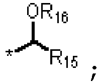 R₆ is -NR₁₁R₁₂; R₁₁ and R₁₂ each independently may be hydrogen, (C1-C7)alkyl, (C3-C6)cycloalkyl, (C6-C10)aryl, heterocycloalkyl(C6-C12)aryl, heteroaryl, and R₁₁ and R₁₂ are linked to each other via alkylene, oxyalkylene, or aminoalkylene to form a 5- or 6-membered heterocyclic ring; R₁₆ may be selected from hydrogen, (C1-C7)alkyl, (C3-C6)cycloalkyl, or (C6-C10)ary; and
(C6-C12)aryl of R₂ and R₆ and heterocyclic ring formed from R₁₁ and R₁₂ may be further substituted with halogen, hydroxyl, (C1-C7)alkyl, (C3-C6)cycloalkyl, 5- or 6-membered heterocycloalkyl, 5- or 6-membered heteroaryl, 5- or 6-membered heterocycloalkylcarbonyl, (C6-C10)aryl (C6-C10)arylcarbonyl, (C1-C6)alkoxy, (C1-C7)alkoxycarbonyl, (C1-C7)alkylcarbonyl, (C3-C6)cyclo(C1-C7)alkylcarbonyl, (C6-C10)ar(C1-C7)alkylcarbonyl, (C3-C6)heteroarylcarbonyl, 5- or 6-membered cycloalkylsulfonyl, (C1-C7)alkylsulfonyl, amino(C1-C7)alkylcarbonyl, (C1-C7)alkyloxycarbonyl, (C6-C10)ar(C1-C7)alkyloxycarbonyl, amino, (C1-C7)alkylamino, (C1-C7)alkylamido, (C1-C7)alkylcarbamoyl, (C1-C7)alkylsulfonamido, or 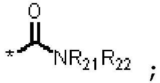 R₂₁ and R₂₂ each may be independently (C1-C7)alkyl, (C3-C6)cycloalkyl, or (C6-C12)ary, or R₂₁ and R₂₂ may be linked to each other via alkylene to form 5- or 6-membered heterocyclic ring.]

Preferably, the compound of Chemical Formula 1, which is the heterocyclic compound according to the present invention, may include imidazopyridine compounds represented by the following Chemical Formula 4.

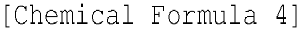

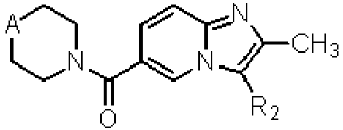

[In Chemical Formula 4,
R₂ is amino, (C6-C10)aryl, or (C6-C10)ar(C1-C7)alkyl;
A is -CH₂ or -NR₃₁; R₃₁ may be selected from hydrogen, (C1-C7)alkyl, (C1-C7)alkylcarbonyl, and (C3-C6)cycloalkylcarbonyl; aryl and arylalkyl of R₂ may be further substituted with halogen, hydroxy, amino, (C1-C7)alkyl, (C3-C6)cycloalkyl, (C1-C6)alkoxy, 5- or 6-membered heterocycloalkyl, or 5- or 6-membered heteroaryl.]

The hetero compound represented by Chemical Formula 1 according to the present invention may be selected from compounds of the following structures, but is not limited thereto.

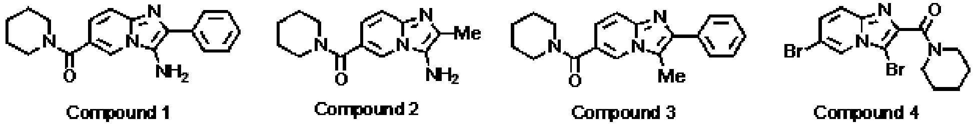

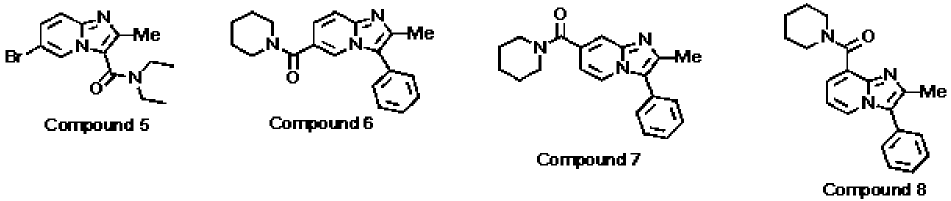

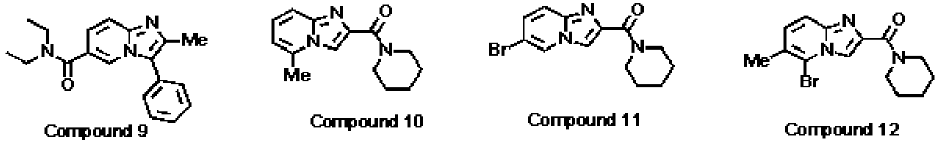

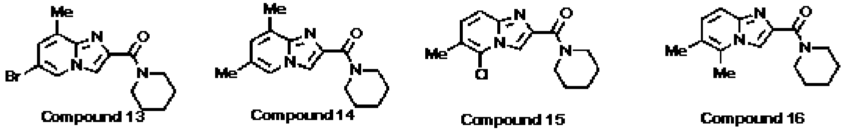

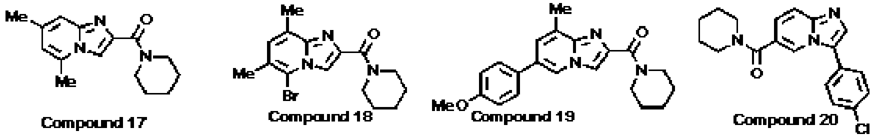

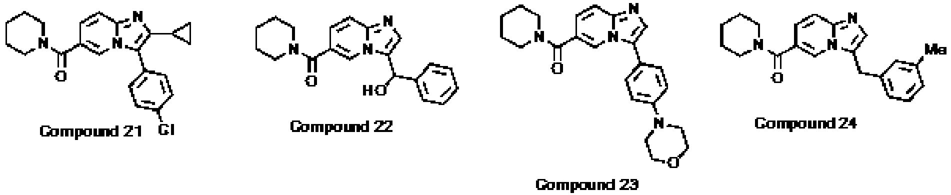

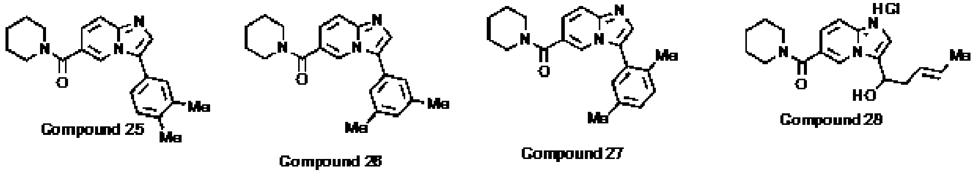

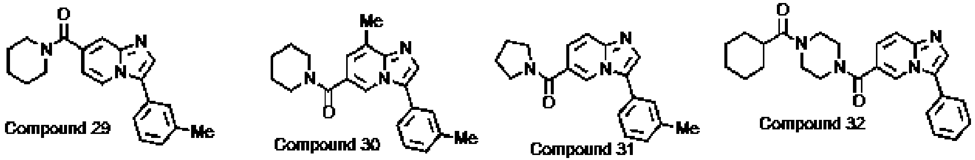

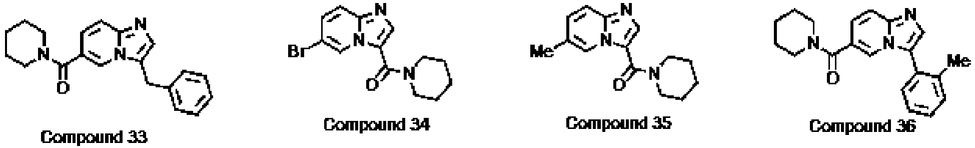

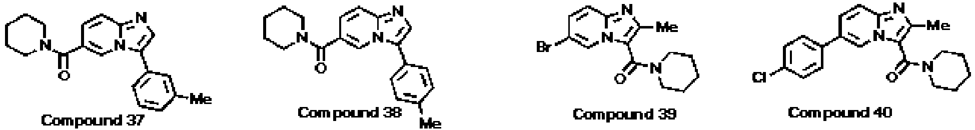

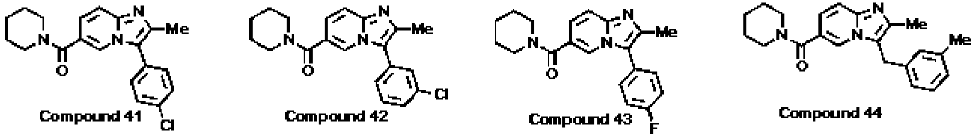

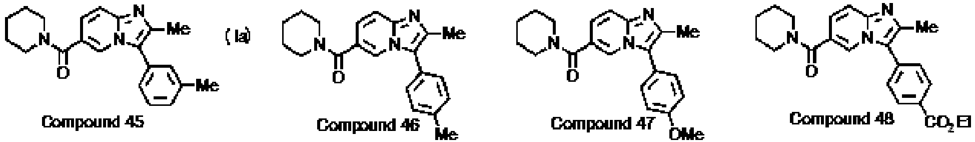

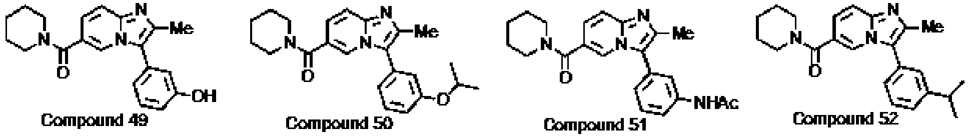

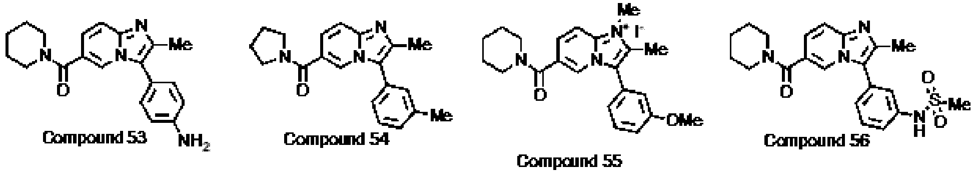

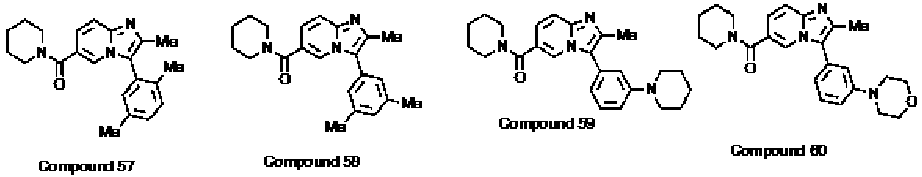

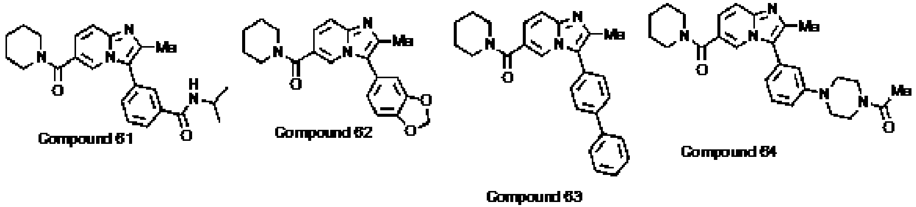

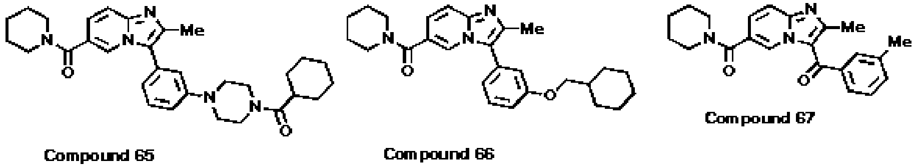

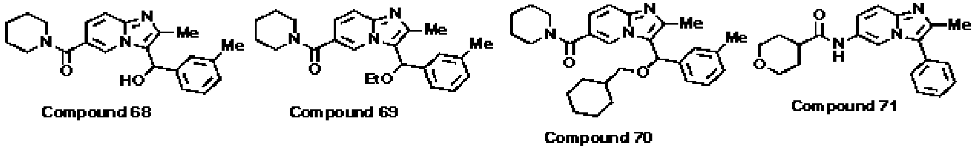

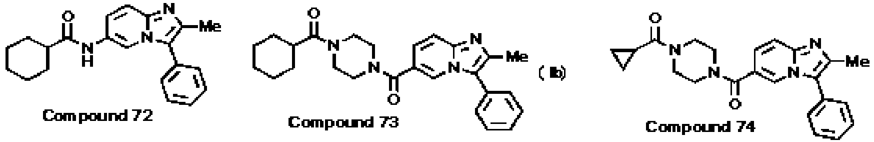

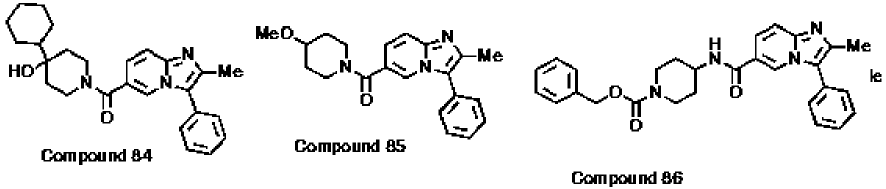

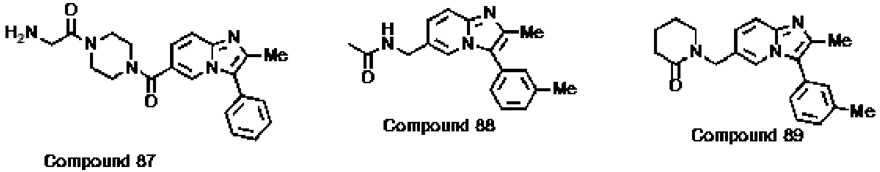

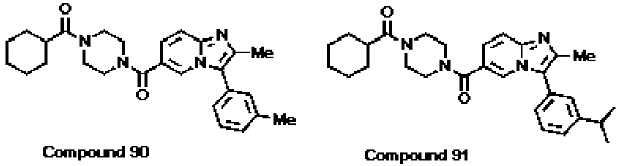

In another general aspect, there is provided a pharmaceutical composition for treating and preventing inflammatory skin diseases containing a heterocyclic compound of Chemical Formula 1 according to the present invention or pharmaceutically acceptable salts thereof.

The inflammatory skin disease may include a skin disease with inflammation and may include acne, rosacea, seborrheic dermatitis, atopic dermatitis, post-inflammatory hyper-pigmentation (PIH), contact dermatitis, pruritus, psoriasis, Lichen planus, eczema, skin infections, Netherson Syndrome, or the like.

In addition, there is provided a pharmaceutical composition for skin wound healing containing a heterocyclic compound of Chemical Formula 1 according to the present invention or pharmaceutically acceptable salts thereof.

The skin wound may include a cut, a stab, an abrasion, a laceration, a bite wound, or the like, but is not limited thereto.

In another general aspect, there is provided a pharmaceutical composition for treating and preventing metastasis of cancer, gastrointestinal disease, asthma, or hepatic cirrhosis containing a heterocyclic compound of Chemical Formula 1 or pharmaceutically acceptable salts thereof according to the present invention.

In addition, there is provided a protease activated receptor-2 (PAR-2) inhibitor composition containing a heterocyclic compound of Chemical Formula 1 according to the present invention or pharmaceutically acceptable salts thereof.

In addition, there is provided a cosmetic composition containing a heterocyclic compound of Chemical Formula 1 according to the present invention.

### [Advantageous Effects]

A compound of Chemical Formula 1, or salts thereof according to the present invention may have a PAR-2 inhibitory activity. It is known that the PAR-2 plays important roles mainly in diseases such as inflammation, cardiovascular diseases, cancer, particularly, metastasis of cancer, gastrointestinal disease such as inflammatory bowel disease (IBD), and the like, asthma, hepatic cirrhosis, or the like. Since the diseases in association with PAR-2 are mostly intractable chronic diseases as described above, the development of an effective PAR-2 inhibitor may be expected to have a large commercial potential.

It was known that particularly, in the skin, the PAR-2 is involved in an important response such as intracellular hyper-pigmentation, induction of pruritus, and the like, as well as inflammation. In addition, it was reported that the PAR-2 plays important roles in maintenance of a skin barrier function, wound healing, and the like. Therefore, the PAR-2 inhibitor is likely to effectively treat skin diseases. In the case of atopic dermatitis in which various skin symptoms are shown at the same time, since in the atopic dermatitis, which is an intractable chronic inflammatory skin disease, various symptoms such as severe pruritus, skin barrier damage, inflammation post-inflammatory hyper-pigmentation (PIH), and the like, that may be expressed by activation of PAR-2 are shown at the same time, the PAR-2 inhibitor may be primarily applied to the atopic disease.

In addition, it was reported that expression of the PAR-2 in the skin wounds is increased, and in the case in which these wounds are treated with a component having the PAR-2 inhibitory activity, the wound healing is stimulated.

Therefore, the compounds of Chemical Formula 1 or salts thereof according to the present invention may be used in order to heal skin wounds as well as to treat and prevent inflammatory diseases such as skin inflammation, atopic dermatitis, or the like and be useful as medicine or a cosmetic composition.

Further, the compound of Chemical Formula 1 and salts thereof according to the present invention may be useful as the PAR-2 inhibitory activity.

### [Description of Drawings]

The above and other objects, features and advantages of the present invention will become apparent from the following description of preferred embodiments given in conjunction with the accompanying drawings, in which:
FIGS 1 and 2 are profiles of a PAR-2 inhibitory activity assay specified in Experimental Example 1;

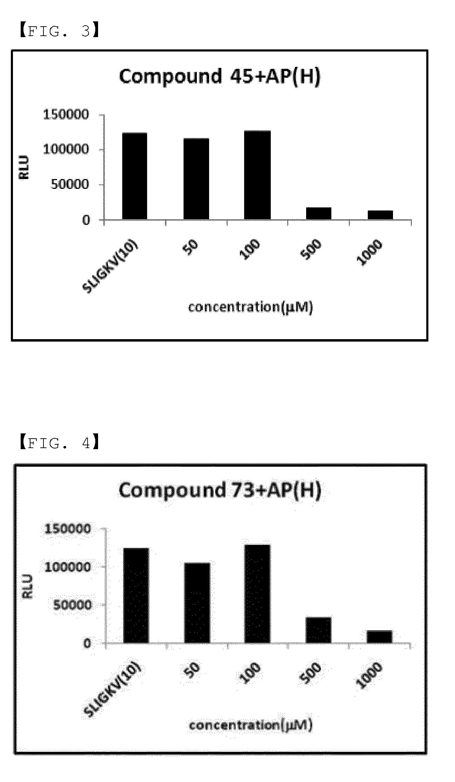

FIGS. 3 and 4 are results of β-arrestin assay in Experimental Example 2;
FIG. 5 is a graph showing an effect of recovering trans-epidermal water loss (TEWL) in oxazolone induced chronic dermatitis animal model;
FIG. 6 is a graph showing an effect of recovering skin hydration in stratum cornea in the oxazolone induced chronic dermatitis animal model; and
FIG. 7 is a graph showing an effect of decreasing a skin thickness in the oxazolone induced chronic dermatitis animal model.

### [Best Mode]

Hereinafter, the present invention will be described in detail.

The present invention provides imidazopyridine compounds represented by the following Chemical Formula 1, which are novel heterocyclic compounds.

### [Chemical Formula 1]

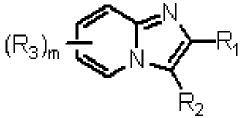

[In Chemical Formula 1,
R₁ is selected from hydrogen, (C1-C7)alkyl, (C3-C6)cycloalkyl, (C6-12)aryl, (C6-C12)ar(C1-C7)alkyl, and 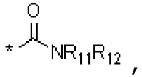 R₂ and R₃ each are independently selected from hydrogen, halogen, (C1-C7)alkyl, (C1-C7)alkoxy, (C1-C7)alkoxycarbonyl, (C3-C6)cycloalkyl, (C6-C10)aryl, (C6-C10)ar(C1-C7)alkyl, (C1-C7) alkylamido(C1-C7)alkyl, -NR₁₁R₁₂, 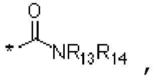 and 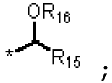 R₁₁, R₁₂, R₁₃, R₁₄, and R₁₅ each are independently selected from hydrogen, (C1-C7)alkyl, (C3-C6)cycloalkyl, (C6-C10)aryl, heterocycloalkyl(C6-C12)aryl, and heteroaryl; R₁₁ and R₁₂, and R₁₃ and R₁₄ are linked to each other via alkylene, oxyalkylene, or aminoalkylene to form a 5- to 6-membered heterocyclic ring; R₁₆ is selected from hydrogen, (C1-C7)alkyl, (C3-C6)cycloalkyl, or (C6-C10)aryl;
m is an integer of 1 to 4,
(C6-C12)aryl of R₂ and R₃ and heterocyclic ring formed from R₁₁ and R₁₂, and R₁₃ and R₁₄ may be further substituted with halogen, hydroxyl, (C1-C7)alkyl, (C3-C6)cycloalkyl, 5- or 6-membered heterocycloalkyl, 5- or 6-membered heteroaryl, 5- or 6-membered heterocycloalkylcarbonyl, (C6-C10)aryl (C6-C10)arylcarbonyl, (C1-C6)alkoxy, (C1-C7)alkoxycarbonyl, (C1-C7)alkylcarbonyl, (C3-C6)cyclo(C1-C7)alkylcarbonyl, (C6-C10)ar(C1-C7)alkylcarbonyl, (C3-C6)heteroarylcarbonyl, 5- or 6-membered cycloalkylsulfonyl, (C1-C7)alkylsulfonyl, amino(C1-C7)alkylcarbonyl, (C1-C7)alkyloxycarbonyl, (C6-C10)ar(C1-C7)alkyloxycarbonyl, amino, (C1-C7)alkylamino, (C1-C7)alkylamido, (C1-C7)alkylcarbamoyl, (C1-C7)alkylsulfonamido, or 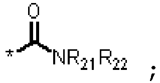 R₂₁ and R₂₂ each are independently (C1-C7)alkyl, (C3-C6)cycloalkyl, or (C6-C12)ary, or R₂₁ and R₂₂ are linked to each other via alkylene to form 5- or 6-membered heterocyclic ring.]

In the present invention, preferable heterocyclic compounds are imidazopyridine compounds represented by the following Chemical Formulas 1a and 1b among imidazopyridine derivative compounds represented by Chemical Formula 1.

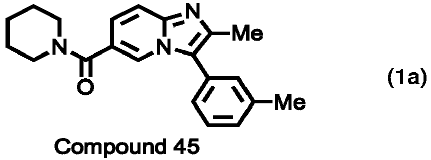

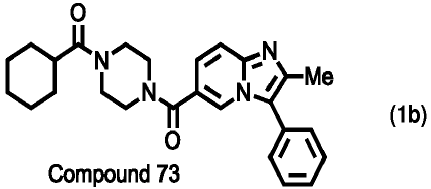

In order to evaluate the inhibitory activity of the compounds of Chemical Formula 1 against the PAR-2 that is known as to be associated with treatment and prevention of skin barrier damage, skin inflammation, psoriasis, Netherson Syndrome, atopic dermatitis, that are inflammatory skin diseases, in vitro and in vivo experiments were performed. As a result, it was confirmed that the compounds according to the present invention have the PAR-2 inhibitory activity in the cell.

Therefore, the present invention provides the pharmaceutical composition for treating or preventing the inflammatory skin diseases, the cosmetic composition, and the PAR-2 inhibitor composition containing the compounds of Chemical Formula 1 or pharmaceutically acceptable salts thereof, wherein the inflammatory skin diseases includes skin barrier damage, psoriasis with skin inflammation, Netherson Syndrome, and atopic dermatitis. In addition, the present invention may provide a composition for skin wound healing using the fact that inhibition of PAR-2 expression may stimulate wound healing.

In the present invention, the composition or the PAR-2 inhibitor may include 0.001 to 90 weight%, more preferably, 0.001 to 50 weight% of the compounds of Chemical Formula 1 according to the present invention as an active ingredient.

Further, the pharmaceutical composition containing the compounds of Chemical Formula 1 or pharmaceutically acceptable salts thereof for treating or preventing the inflammatory skin diseases or for skin wound healing and the PAR-2 inhibitor composition according to the present invention may include all of the compositions that may be administered to a human for skin external application, injection, inhalation, oral administration, and the like. Particularly, a formulation that may be applied to the skin or mucosa is not particularly limited, but the composition may be formulated as a solution, emulsion, suspension, cream, ointment, gel, jelly, or spray.

The compound of Chemical Formula 1 according to the present invention may be prepared through the pathway of the following Reaction Formula 1.

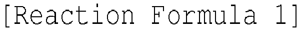

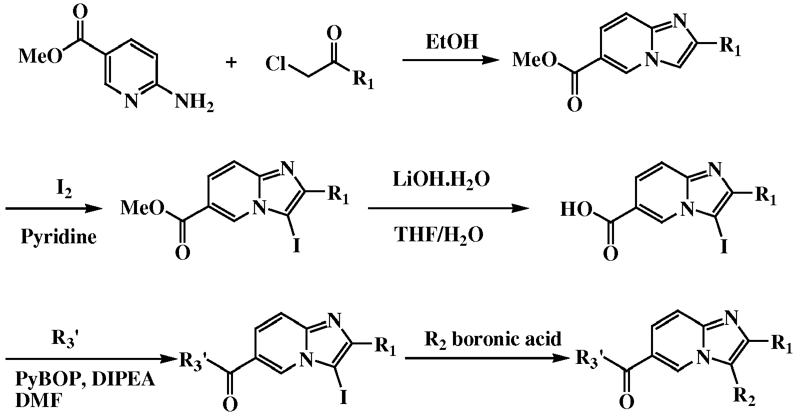

Hereinafter, the present invention will be described in detail through the following Examples and Experimental Examples, but is not limited thereto.

### Example 1: Preparation of 2-methyl-3-m-tolylimidazo[1,2-a]pyridin-6-yl)(piperidin-1-yl) methanone (Compound 45)

### [Step 1] Preparation of methyl 2-methylimidazo[1,2-a]pyridine-6-carboxylate

6-aminonicotinic acid methylester (500mg, 3.29mmol) and chloroacetone (1.83g, 19.74mmol) were dissolved in ethanol (15mL), followed by refluxing and stirring for 24 hours. After removing a solvent, the resultant was diluted with water (15mL), basified by sodium hydrogen carbonate saturated aqueous solution and then extracted three times with methylene chloride (MC, 10mL). The organic layer was collected, dried over anhydrous magnesium sulfate, concentrated under reduce pressure, and purified by column chromatography (MC: MeOH = 40:1), thereby obtaining methyl 2-methylimidazo[1,2-a]pyridine-6-carboxylate (340mg, 54%).

¹H NMR (600MHz, chloroform-d1) d=8.80(s, 1H), 7.65(d, J=9.6Hz, 1H), 7.45(d, J=9.6Hz, 1H), 7.38(s, 1H)113, 3.91(s, 3H), 2.45(s, 3H)

### [Step 2] Preparation of methyl 3-iodo-2-methylimidazo[1,2-a]pyridine-6-carboxylate

The compound (340mg, 1.79mmol) obtained in step 1 was dissolved in pyridine (4mL), and I2 (681mg, 2.68mmol) was added thereto, followed by stirring at 50°C for 4 hours. After water (80mL) was added to the mixture, and the resultant material was extracted three times with MC (50mL). The organic layer was collected, dried over anhydrous magnesium sulfate, concentrated under reduce pressure, and purified by column chromatography (MC: EA = 3:1). Then, the concentrated compound was washed with n-hex (100mL) and concentrated under reduced pressure, thereby obtaining methyl 3-iodo-2-methylimidazo[1,2-a]pyridine-6-carboxylate (470m, 83%).

¹H NMR (600MHz, chloroform-d1) d=8.79(s, 1H), 7.74(d, J=9.6Hz, 1H), 7.49(d, J=9.6Hz, 1H), 1133.96(s, 3H), 2.50(s, 3H)

### [Step 3] Preparation of 3-iodo-2-methylimidazo[1,2-a]pyridine-6-carboxylic acid

3-iodo-2-methylimidazo[1,2-a]pyridine-6-carboxylic acid (202mg, 85%) was obtained using the compound (250mg, 0.791mmol) obtained in step 2 by the same method as that in Preparation Example 6-4. The obtained compound was directly used in a next reaction without purification.

### [Step 4] Preparation of (3-iodo-2-methylimidazo[1,2-a]pyridin-6-yl)(piperidin-1-yl) methanone

(3-iodo-2-methylimidazo[1,2-a]pyridin-6-yl)(piperidin-1-yl) methanone (180mg, 73%) was obtained using the compound (202mg, 0.669mmol) obtained in step 3 by the same method as that in Preparation Example 6-5.

¹H NMR (600MHz, chloroform-d1) d=8.25(s, 1H), 7.49(d, J=9.0Hz, 1H), 7.21(d, J=9.6Hz, 1H), 3.71(br d, 4H), 1132.50 (s, 3H), 1,71(m, 6H)

### [Step 5] Preparation of 2-methyl-3-m-tolylimidazo[1,2-a]pyridin-6-yl)(piperidin-1-yl) methanone (Compound 45)

The compound (20mg, 0.054mmol) obtained in step 4, 3-methylphenylboronic acid (30.6mg, 0.065mmol), and tetrakis(triphenylphosphine) palladium (40 mg, 0.035mmol) were dissolved in DME (6mL), and then potassium carbonate aqueous solution (696µL, 2M) was added thereto, followed by refluxing and stirring for 20 hours. After the mixture was cooled to room temperature and MC (10ml) was added thereto, the resultant was washed with water (5mL). The organic layer was dried over anhydrous magnesium sulfate, concentrated under reduce pressure, and purified by column chromatography (MC: EA = 3:1), thereby obtaining Compound 45 (14mg, 77%).

¹H NMR (600MHz, chloroform-d1) d =8.28(s, 1H), 7.56(d, J=10.8Hz, 1H), 7.46(t, J=7.2Hz, 1H, 7.27(m, 3H), 7.16(dd ,J=8.4Hz, 1.2Hz, 1H), 3.65(br d , 4H), 2.48(s, 3H), 2.44(s, 3H), 1.69(m 6H)

### Example 2: Preparation of (4-(cyclohexanecarbonyl)piperazin-1-yl)(2-methyl-3-phenyl imidazo[1,2-a]pyridin-6-yl)methanone (Compound 73)

### [Step 1] Preparation of methyl 2-methyl-3-phenylimidazo[1,2-a]pyridine-6-carboxylate

The compound (220mg, 0.696mmol) obtained in step 2 of Example 1, phenylboronic acid (94mg, 0.77mmol), and tetrakis(triphenylphosphine) palladium (40mg, 0.035mmol) were dissolved in DME (6mL), and then potassium carbonate aqueous solution (696µL, 2M) was added thereto, followed by refluxing and stirring for 20 hours. After the mixture was cooled to room temperature and MC (10ml) was added thereto, the resultant was washed with water (5mL). The organic layer was dried over anhydrous magnesium sulfate, concentrated under reduce pressure, and purified by column chromatography (MC: EA = 3:1), thereby obtaining methyl 2-methyl-3-phenylimidazo[1,2-a]pyridine-6-carboxylate (80mg, 43%).

¹H NMR (600MHz, chloroform-d1) d=8.83 (s, 1H), 7.71∼7.47(m,7H), 113, 3.90(s, 3H), 2.50(s, 3H)

### [Step 2] Preparation of 2-methyl-3-phenylimidazo[1,2-a]pyridine-6-carboxylic acid

After the compound (80mg, 0.30mmol) obtained in step 1 was dissolved in a mixture of tetrahydrofuran (2mL) and ethanol (2mL), lithium hydroxide (20mg, 0.48mmol) was added thereto, and water (1mL) was added thereto, followed by stirring for 12 hours. The resultant was neutralized with 1N HCl and concentrated under reduced pressure, thereby obtaining 2-methyl-3-phenylimidazo[1,2-a]pyridine-6-carboxylic acid (78mg, 98%). The obtained compound was directly used in a next reaction without purification

### [Step 3] Preparation of tert-butyl 4-(2-methyl-3-phenylimidazo[1,2-a]pyridine-6-carbonyl)piperazine-1-carboxylate

The compound (100mg, 0.396mmol) obtained in step 2 and tert-butyl piperazine-1-carboxylate (89mg, 0.475mmol) were dissolved in DMF (2mL), DIPEA (253µL, 1.452mmol), N-Boc piperazine (90mg, 1.057mmol) were sequentially added thereto. Then, PyBOP (259 mg, 498mmol) was added thereto, followed by stirring at room temperature for 1 hour. After water (10mL) was added to the reaction mixture, the resultant was extracted with ethylacetate (12mL). The organic layer was washed with saturated saline (10mL), dried over anhydrous magnesium sulfate, concentrated under reduced pressure, and purified by column chromatography (MC: EA =1:1 to 1:2), thereby obtaining tert-butyl 4-(2-methyl-3-phenylimidazo[1,2-a]pyridine-6-carbonyl)piperazine-1-carboxylate (100mg, 60%).

¹H NMR (600MHz, chloroform-d1) d =8.33(s, 1H), 7.59∼7.44(m, 6H), 7.16(dd ,J=7.2Hz, 1.2Hz, 1H), 3.86(br s, 4H), 3.38(br s, 4H), 2.53(s, 3H)

### [Step 4] Preparation of (2-methyl-3-phenylimidazo[1,2-a]pyridin-6-yl)(piperazin-1-yl) methanone

HCl dioxane (5mL, 4M) was added to the compound (100mg, 0.238mmol) obtained in step 3, followed by stirring at room temperature for 2 hours. The mixture was concentrated under reduced pressure, thereby obtaining (2-methyl-3-phenylimidazo[1,2-a]pyridin-6-yl)(piperazin-1-yl) methanone hydrochloride salts (108mg, 99%).

¹H NMR (600MHz, CD30D-d4) d=8.62(s, 1H), 8.02(m, 2H), 7.69(m, 5H), 3.59(br s, 4H), 3.46(br s, 4H), 2.50(s, 3H), 1.47(s, 9H)

### [Step 5] Preparation of (4-(cyclohexanecarbonyl)piperazin-1-yl)(2-methyl-3-phenyl imidazo[1,2-a]pyridin-6-yl)methanone (Compound 73)

After the compound (27mg, 0.076mmol) obtained in step 4 was dissolved in MC(1mL), TEA (32µL, 0.228mmol) was added thereto, and then cyclohexanecarbonylchloride (12µL, 0.091mmol) was added thereto at 0°C. Stirring was performed at 0°C for 10 minutes and again at room temperature for 30 minute, and then methanol (0.1mL) was added thereto. The resultant was concentrated under reduce pressure, and purified by column chromatography (MC: MeOH = 40:1), thereby obtaining Compound 73 (26mg, 79%).

¹H NMR (600MHz, chloroform-d1) d=8.34(s, 1H), 7.59∼7.45(m, 6H), 7.16(dd, J=9.0Hz, 1.2Hz, 1H), 3.63(br d, 8H), 2.47(s, 3H), 1.90∼1.24(m 11H)

In order to evaluate the PAR-2 inhibitory effect of Compounds 45 and 73 prepared in Examples 1 and 2, in vitro and in vivo experiments were performed.

### <Evaluation of in vitro activity of PAR-2 inhibitor>

### Experimental Example 1: Calcium Immobilization Assay

In order to evaluate inhibitory activity of the synthesized materials against the PAR-2, primarily, an assay system in which a concentration of calcium ions in cell may be measured in real time was constructed, and evaluation was conducted using this system. The PAR-2 is a kind of G-protein coupled receptors (GPCR), and when the PAR-2 is activated in cell, phospholipase C-b (PLC-b) decomposes phosphatidylinositol 4,5-bisphosphate (PIP2) into inositol triphosphate (IP3) and diacyl glycerol (DAG) while being activated. IP3 generated at this time stimulates secretion of calcium ions in endoplasmic reticulum (ER), which is a storage of calcium ions in a cell, such that the concentration of calcium ions in the cell is increased. Based on an existing study result, an in vitro screening assay system capable of detecting a material capable of suppressing the concentration of calcium ions in the cell from being increased by activation of PAR-2 was constructed. As a PAR-2 activating agent, activating peptide (AP) was used, and as a reagent for detecting the concentration of calcium ion in the cell, a Calcium-4 assay kit produced (Molecular Probe). As a cell line used in experiments, HCT-15 in which PAR-2 is over- expressed was used. Inhibitor candidates were treated for 5 minutes and then treated with AP, a change in fluorescence was observed for 2 minutes in real time using a FlexStation II (Molecular Device Corp.).

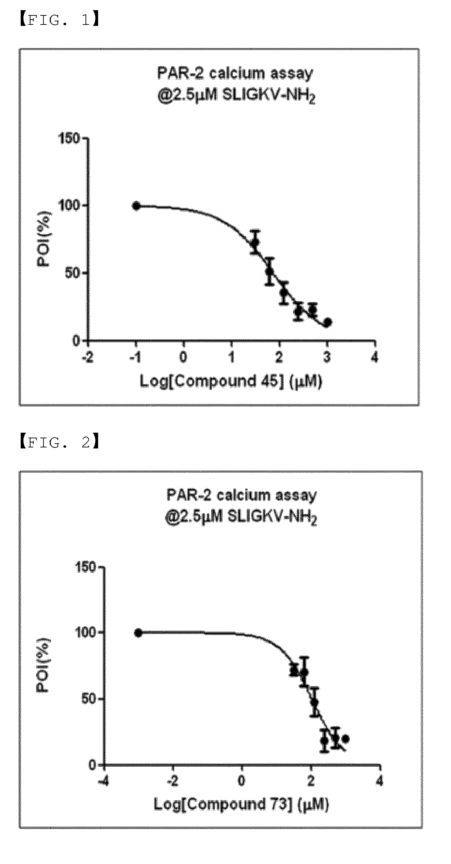

The inhibitory activity of the synthesized materials against the PAR-2 was evaluated using the protocol as described above, and profiles of the assay for Compounds 45 and 73 prepared in Examples 1 and 2 were shown in FIGS. 1 and 2.

### Experimental Example 2: β-Arrestin GPCR Assay

An in vitro assay protocol capable of detecting activation of the PAR-2 was additionally introduced in addition to a calcium immobilization assay to measure the activity of the selected materials. First, the PAR-2 inhibitory activities of two kinds of samples were measured using a PathHunterTM β-arrestin GPCR assay kit (DiscoveRx Corp.) capable of detecting accumulation of β-arrestin, which is an intracellular reaction firstly generated by the activation of the PAR-2. Experiments were conducted by diluting concentrations of the samples by one-half, ranging from 1000 to 50 µM, and SKIGKV-NH2 (10µM) was treated with the PAR-2 inhibitor. As an experimental result, suppression of β-arrestin was confirmed in both of the two kinds of samples at a concentration of 500µM. As a measurement result, it was observed that IC50 values were different from those in calcium immobilization assay (See Table 1).

**Table 1. Result comparison of Calcium-4 assay and β-arrestin assay**

| Compound | IC50 (µM) | | CC50 (µM) |
|---|---|---|---|
| | Calcium-4 | PathHunter™ | |
| Compound 45 | 73.79 | 338.56 | > 100 |
| Compound 73 | 76.68 | 400.78 | > 100 |

### Experimental Example 3: Confocal Microscopy

In order to visually confirm the activation of the PAR-2, a cell line having the following structure was prepared based on existing references, and activation of the PAR-2 was observed using a confocal microscope. As the cell line, Kirsten Murine Sarcoma Virus transformed rat kidney epithelial cell (KNRK cell) was used. In this cell line, the PAR-2 may not be expressed in a normal state. Each of the N-terminals of KNRK cell were tagged with Flag epitope, and each of the C-terminals were tagged with Myc epitope, such that a stable transfected cell was constructed. Each of Flag and Myc with respect to the KNRK-PAR-2 cell line prepared as described above was double-stained, and whether or not the PAR-2 was activated was observed using the confocal microscope ([trypsin]=1µM, [SLIGKV(AP)]=100µM). In a state in which the PAR-2 was not activated, Flag and Myc were double stained, such that a yellow image was shown in a merged image. However, in the case in which the PAR-2 was activated, a red image was shown while the Flag is separated.

In the case in which the KNRK-PAR-2 cell line was simultaneously treated with trypsin, activating peptide (SLIGKV), and the selected inhibitor material, Compound 45 ([trypsin]=1µM, [SLIGKV (activating peptide)]=100µM, [Compound 45]=200µM), it was observed that a yellowish image was shown, such that it was confirmed that activation of the PAR-2 was suppressed.

### <Evaluation of in vivo activity of PAR-2 inhibitor>

### Experimental Example 4: oxazolone model

Effects of the selected materials were evaluated using chronic dermatitis animal model. Oxazolone induced chronic dermatitis animal model by continuously applying oxazolone, which is a kind of hapten, to a hairless mouse was used. It was reported that various symptoms of atopic dermatitis relatively accurately appear in recently developed oxazolone model, and the present inventors also confirmed through the prior study that various symptoms of general atopic dermatitis appear similarly to clinically appearing symptoms. In order to construct the chronic dermatitis animal model, 5% oxazolone was applied and sensitized to the hairless mice (SKH-1, 6 weeks old). After 1 week, 0.1% oxazolone was applied every other day for 2 weeks, thereby induced the symptoms of dermatitis. After samples were applied to the dermatitis induced animal once a day for 3 days, changes in various skin functions and a skin thickness were observed. Finally, biopsy was performed on skin tissue, and histological observation was conducted.

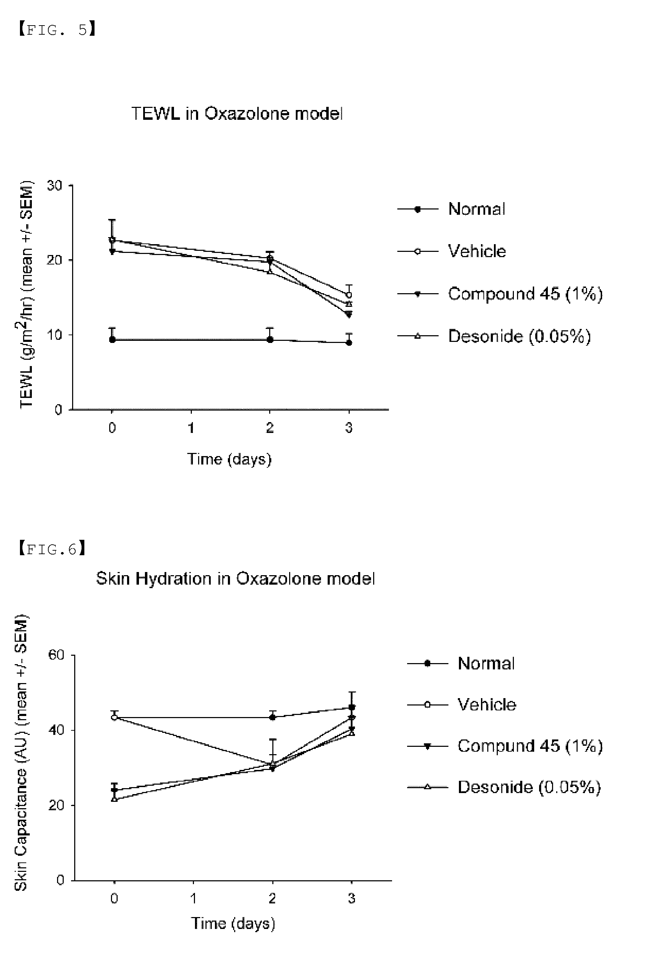

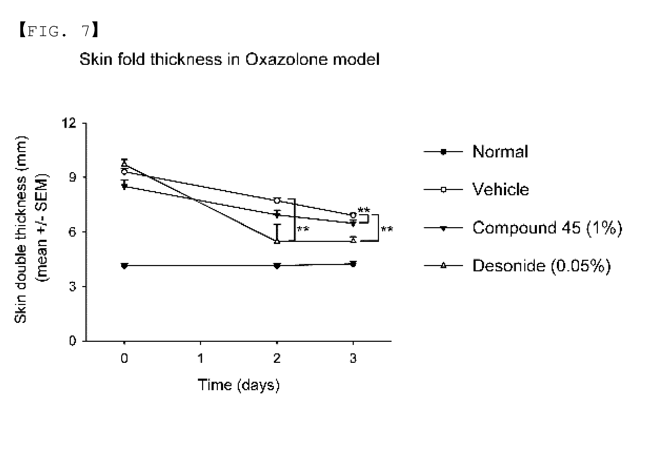

As a positive control group used in the experiment, 0.05% desonide formulation, which is a steroid having relatively weak potency, was used, and changes in trans-epidermal water loss (TEWL) skin hydration in stratum cornea, and skin thickness were observed. As shown in FIGS. 5 to 7, it was observed that Compound 45 may effectively restore trans-epidermal water loss (TEWL) and skin hydration in stratum cornea and reduce the skin thickness. Particularly, Compound 45 has statistically significant improvement effect on the skin thickness.

## Claims

1. Heterocyclic compounds represented by the following Chemical Formula 1. 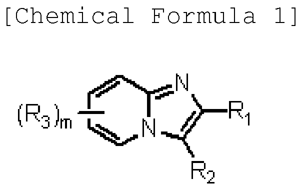 [In Chemical Formula 1,
R₁ is selected from hydrogen, (C1-C7)alkyl, (C3-C6)cycloalkyl, (C6-12)aryl, (C6-C12)ar(C1-C7)alkyl, and 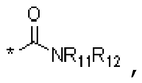 R₂ and R₃ each are independently selected from hydrogen, halogen, (C1-C7)alkyl, amino, (C1-C7)alkoxy, (C1-C7)alkoxycarbonyl, (C3-C6)cycloalkyl, (C6-C10)aryl, (C6-C10)ar(C1-C7)alkyl, (C1-C7) alkylamido(C1-C7)alkyl, -NR₁₁R₁₂, 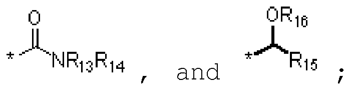 R₁₁, R₁₂, R₁₃, R₁₄, and R₁₅ each are independently selected from hydrogen, (C1-C7)alkyl, (C3-C6)cycloalkyl, (C6-C10)aryl, heterocycloalkyl(C6-C12)aryl, and heteroaryl; R₁₁ and R₁₂, and R₁₃ and R₁₄ are linked to each other via alkylene, oxyalkylene, or aminoalkylene to form a 5- to 6-membered heterocyclic ring; R₁₆ is selected from hydrogen, (C1-C7)alkyl, (C3-C6)cycloalkyl, or (C6-C10)aryl;
m is an integer of 1 to 4; and
(C6-C12)aryl of R₂ and R₃ and heterocyclic ring formed from R₁₁ and R₁₂ and R₁₃ and R₁₄ may be further substituted with halogen, hydroxyl, (C1-C7)alkyl, (C3-C6)cycloalkyl, 5- or 6-membered heterocycloalkyl, 5- or 6-membered heteroaryl, 5- or 6-membered heterocycloalkylcarbonyl, (C6-C10)aryl (C6-C10)arylcarbonyl, (C1-C6)alkoxy, (C1-C7)alkoxycarbonyl, (C1-C7)alkylcarbonyl, (C3-C6)cyclo(C1-C7)alkylcarbonyl, (C6-C10)ar(C1-C7)alkylcarbonyl, (C3-C6)heteroarylcarbonyl, 5- or 6-membered cycloalkylsulfonyl, (C1-C7)alkylsulfonyl, amino(C1-C7)alkylcarbonyl, (C1-C7)alkyloxycarbonyl, (C6-C10)ar(C1-C7)alkyloxycarbonyl, amino, (C1-C7)alkylamino, (C1-C7)alkylamido, (C1-C7)alkylcarbamoyl, (C1-C7)alkylsulfonamido, or 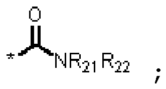 R₂₁ and R₂₂ each are independently (C1-C7)alkyl, (C3-C6)cycloalkyl, or (C6-C12)ary, or R₂₁ and R₂₂ are linked to each other via alkylene to form 5- or 6-membered heterocyclic ring.]

2. The Heterocyclic compounds of claim 1, wherein it is represented by the following Chemical Formula 2. 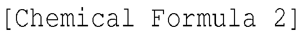 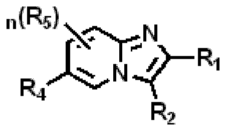 [In Chemical Formula 2,
R₁ is selected from hydrogen, (C1-C7)alkyl, (C3-C6)cycloalkyl, (C6-12)aryl, and 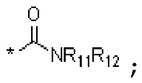 R₂ is independently selected from hydrogen, halogen, amino, (C1-C7)alkyl, (C6-10)aryl, -NR₁₁R₁₂, 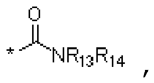 and 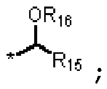 R₄ is selected from hydrogen, halogen, (C1-C7)alkyl, (C1-C7)alkoxy, (C1-C7)alkoxycarbonyl, (C6-C10)aryl, (C1-C7)alkylamido(C1-C7)alkyl, -NR₁₁R₁₂, 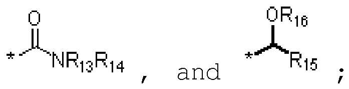 R₁₁, R₁₂, R₁₃, R₁₄, and R₁₅ each are independently selected from hydrogen, (C1-C7)alkyl, (C3-C6)cycloalkyl, (C6-C10)aryl, heterocycloalkyl(C6-C12)aryl, and heteroaryl; R₁₁ and R₁₂, and R₁₃ and R₁₄ are linked to each other via alkylene, oxyalkylene, or aminoalkylene to form a 5- to 6-membered heterocyclic ring; R₁₆ is selected from hydrogen, (C1-C7)alkyl, (C3-C6)cycloalkyl, or (C6-C10)aryl;
(C6-C12)aryl of R₂ and R₄ and heterocyclic ring formed from R₁₁ and R₁₂, and R₁₃ and R₁₄ may be further substituted with halogen, hydroxyl, (C1-C7)alkyl, (C3-C6)cycloalkyl, 5- or 6-membered heterocycloalkyl, 5- or 6-membered heteroaryl, 5- or 6-membered heterocycloalkylcarbonyl, (C6-C10)aryl (C6-C10)arylcarbonyl, (C1-C6)alkoxy, (C1-C7)alkoxycarbonyl, (C1-C7)alkylcarbonyl, (C3-C6)cyclo(C1-C7)alkylcarbonyl, (C6-C10)ar(C1-C7)alkylcarbonyl, (C3-C6)heteroarylcarbonyl, 5- or 6-membered cycloalkylsulfonyl, (C1-C7)alkylsulfonyl, amino(C1-C7)alkylcarbonyl, (C1-C7)alkyloxycarbonyl, (C6-C10)ar(C1-C7)alkyloxycarbonyl, amino, (C1-C7)alkylamino, (C1-C7)alkylamido, (C1-C7)alkylcarbamoyl, (C1-C7)alkylsulfonamido, or 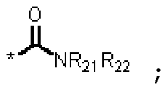 R₂₁ and R₂₂ each are independently (C1-C7)alkyl, (C3-C6)cycloalkyl, or (C6-C12)ary, or R₂₁ and R₂₂ are linked to each other via alkylene to form 5- or 6-membered heterocyclic ring;
R₅ is independently selected from halogen and (C1-C7)alkyl; and
n is an integer of 1 to 3.]

3. The heterocyclic compounds of claim 1, wherein it is represented by the following Chemical Formula 3.
[Chemical Formula 3] 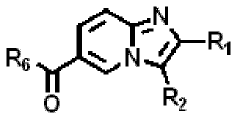 [In Chemical Formula 3,
R₁ is selected from hydrogen, (C1-C7)alkyl, (C3-C6)cycloalkyl, (C6-12)aryl, and 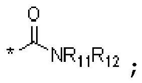 R₂ is independently selected from hydrogen, halogen, amino, (C1-C7)alkyl, (C6-10)aryl, -NR₁₁R₁₂, 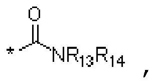 and 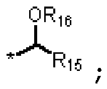 R₆ is -NR₁₁R₁₂; R₁₁ and R₁₂ each are independently hydrogen, (C1-C7)alkyl, (C3-C6)cycloalkyl, (C6-C10)aryl, heterocycloalkyl(C6-C12)aryl, heteroaryl, R₁₁ and R₁₂ are linked to each other via alkylene, oxyalkylene, or aminoalkylene to form a 5- or 6-membered heterocyclic ring; R₁₆ is selected from hydrogen, (C1-C7)alkyl, (C3-C6)cycloalkyl, or (C6-C10)ary; and
(C6-C12)aryl of R₂ and R₆ and heterocyclic ring formed from R₁₁ and R₁₂ may be further substituted with halogen, hydroxyl, (C1-C7)alkyl, (C3-C6)cycloalkyl, 5- or 6-membered heterocycloalkyl, 5- or 6-membered heteroaryl, 5- or 6-membered heterocycloalkylcarbonyl, (C6-C10)aryl (C6-C10)arylcarbonyl, (C1-C6)alkoxy, (C1-C7)alkoxycarbonyl, (C1-C7)alkylcarbonyl, (C3-C6)cyclo(C1-C7)alkylcarbonyl, (C6-C10)ar(C1-C7)alkylcarbonyl, (C3-C6)heteroarylcarbonyl, 5- or 6-membered cycloalkylsulfonyl, (C1-C7)alkylsulfonyl, amino(C1-C7)alkylcarbonyl, (C1-C7)alkyloxycarbonyl, (C6-C10)ar(C1-C7)alkyloxycarbonyl, amino, (C1-C7)alkylamino, (C1-C7)alkylamido, (C1-C7)alkylcarbamoyl, (C1-C7)alkylsulfonamido, or 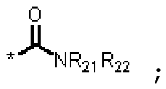 R₂₁ and R₂₂ each are independently (C1-C7)alkyl, (C3-C6)cycloalkyl, or (C6-C12)ary, or R₂₁ and R₂₂ are linked to each other via alkylene to form 5- or 6-membered heterocyclic ring.]

4. The heterocyclic compounds of claim 1, wherein it is represented by the following Chemical Formula 4. 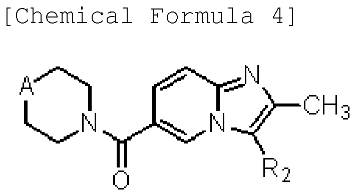 [In Chemical Formula 4,
R₂ is amino, (C6-C10)aryl, or (C6-C10)ar(C1-C7)alkyl;
A is -CH₂ or -NR₃₁; R₃₁ is selected from hydrogen, (C1-C7)alkyl, (C1-C7)alkylcarbonyl, and (C3-C6)cycloalkylcarbonyl; and aryl and arylalkyl of R₂ may be further substituted with halogen, hydroxy, amino, (C1-C7)alkyl, (C3-C6)cycloalkyl, (C1-C6)alkoxy, 5- or 6-membered heterocycloalkyl, or 5- or 6-membered heteroaryl.]

5. The heterocyclic compounds of claim 1, wherein it is selected from compounds of the following structures.

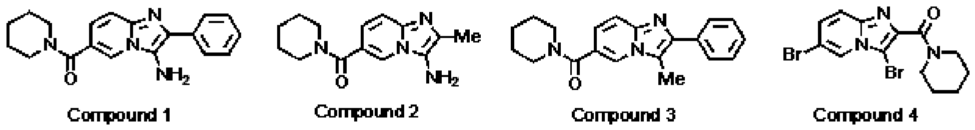

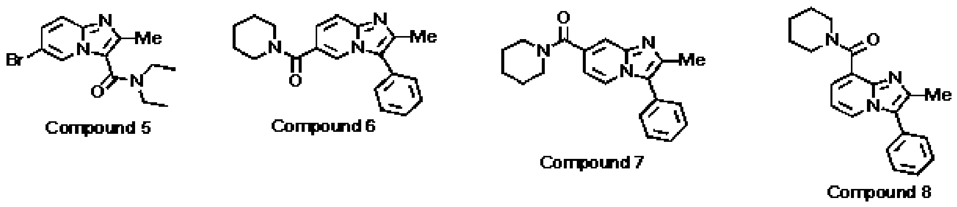

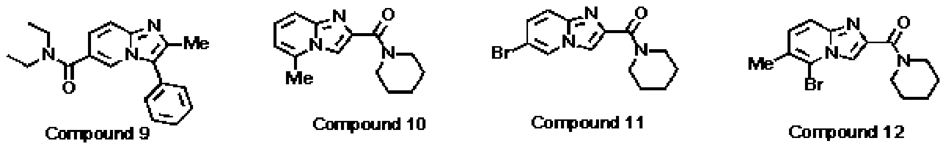

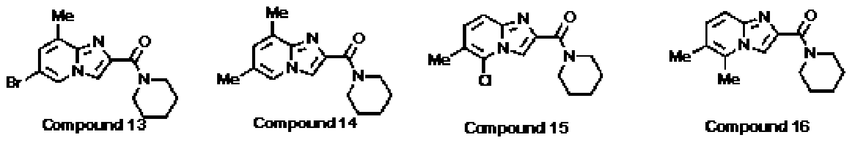

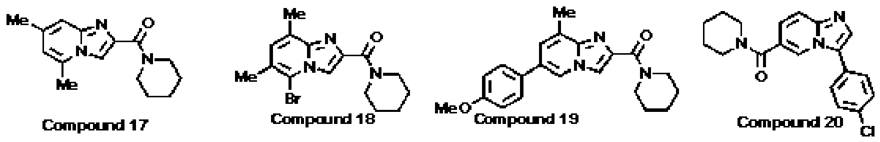

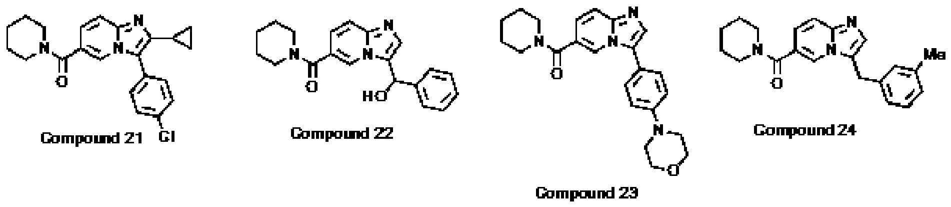

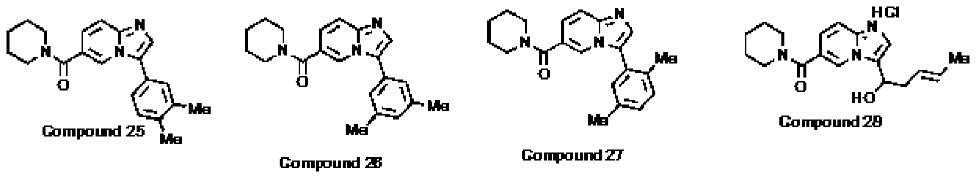

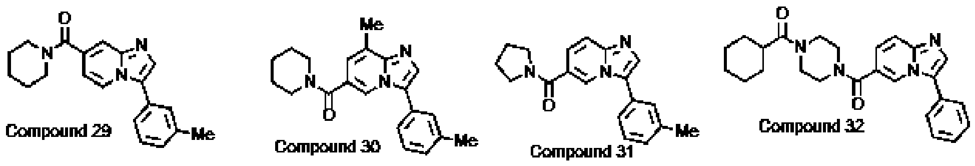

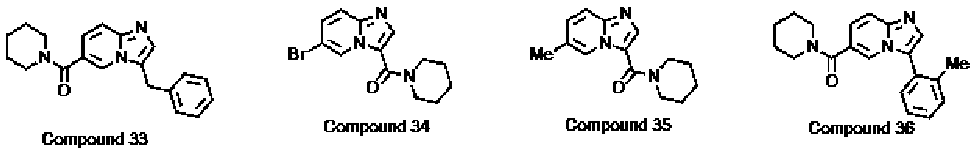

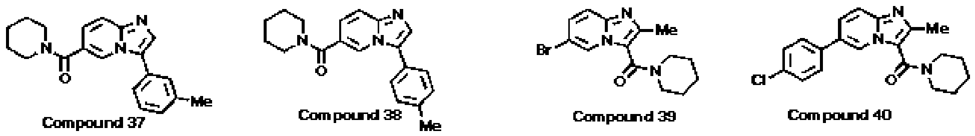

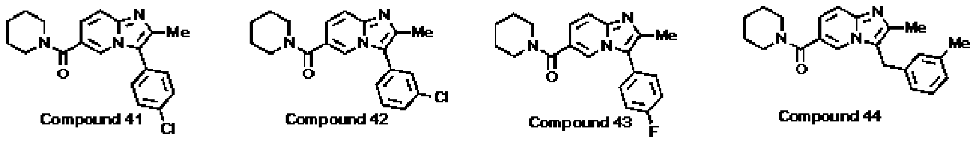

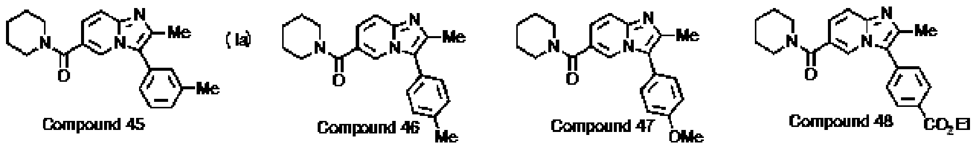

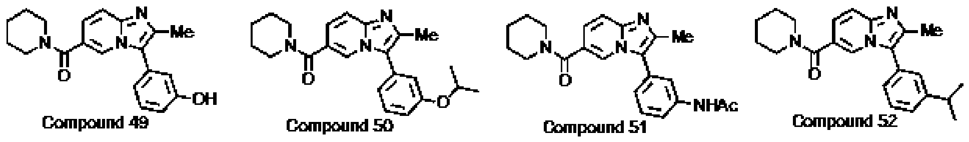

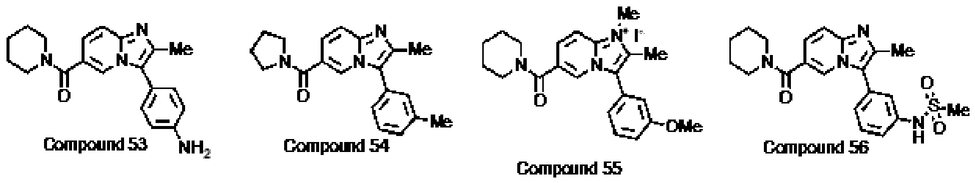

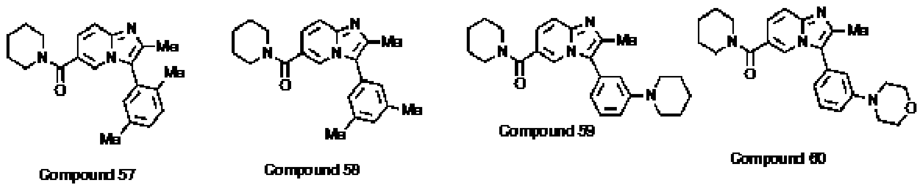

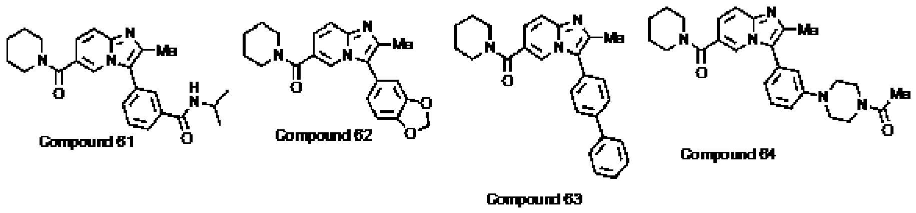

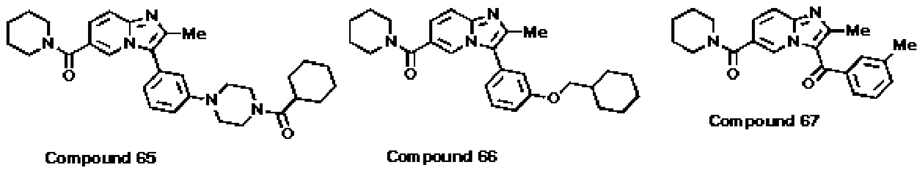

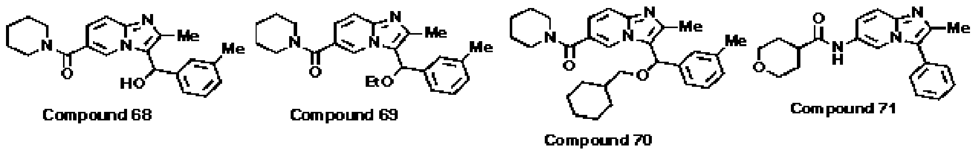

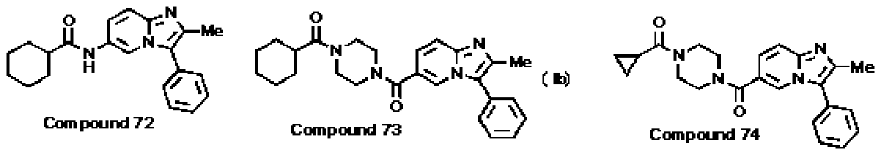

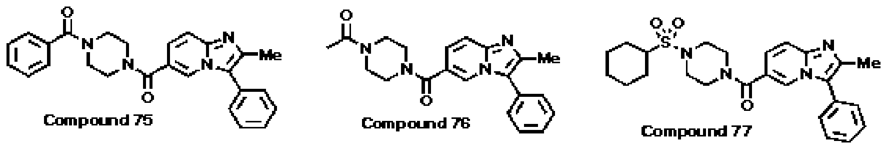

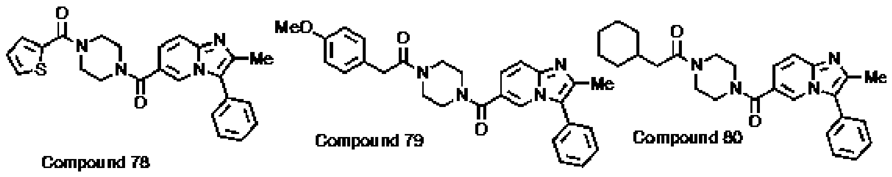

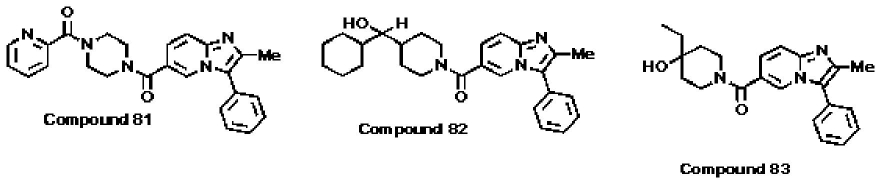

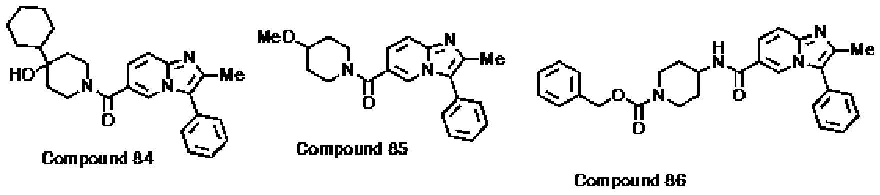

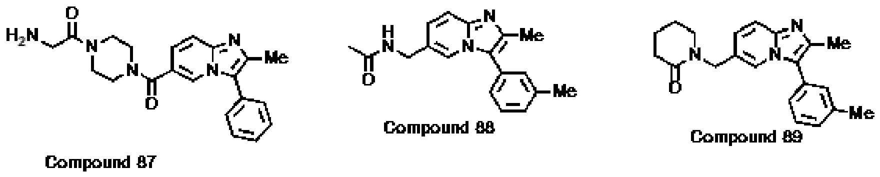

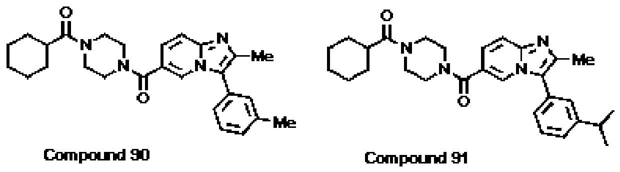

6. A pharmaceutical composition for treating and preventing inflammatory skin diseases comprising the heterocyclic compound of Chemical Formula 1 of claims 1 to 5 or pharmaceutically acceptable salts thereof.

7. The pharmaceutical composition for treating and preventing inflammatory skin diseases of claim 6, wherein the inflammatory skin disease includes a skin disease with inflammation and may include acne, rosacea, seborrheic dermatitis, atopic dermatitis, post-inflammatory hyper-pigmentation (PIH), contact dermatitis, pruritus, psoriasis, Lichen planus, eczema, skin infections, Netherson Syndrome, or the like.

8. A pharmaceutical composition for skin wound healing comprising the heterocyclic compound of Chemical Formula 1 of claims 1 to 5 or pharmaceutically acceptable salts thereof.

9. A pharmaceutical composition for treating and preventing metastasis of cancer, gastrointestinal disease, asthma, or hepatic cirrhosis comprising the heterocyclic compound of Chemical Formula 1 of claims 1 to 5 or pharmaceutically acceptable salts thereof.

10. A protease activated receptor-2 (PAR-2) inhibitor composition comprising the heterocyclic compound of Chemical Formula 1 of claims 1 to 5 or pharmaceutically acceptable salts thereof.

11. A cosmetic composition comprising the heterocyclic compound represented by Chemical Formula 1 of claims 1 to 5.
